(19) Europäisches Patentamt
European Patent Office
Office européen des brevets

(11) Publication number: **0 269 311**
**A2**

(12) # EUROPEAN PATENT APPLICATION

(21) Application number: 87309950.1

(51) Int. Cl.⁴: **A61K 37/64**

(22) Date of filing: 11.11.87

(30) Priority: 17.11.86 US 931489

(43) Date of publication of application: 01.06.88 Bulletin 88/22

(84) Designated Contracting States: CH DE FR GB IT LI NL

(71) Applicant: **MERCK & CO. INC.**
126, East Lincoln Avenue P.O. Box 2000
Rahway New Jersey 07065-0900(US)

(72) Inventor: **Eilon, Gabriel F.**
159 Ardwick Terrace
Lansdale Pennsylvania 19446(US)

(74) Representative: **Hesketh, Alan, Dr.**
European Patent Department Merck & Co., Inc. Terlings Park Eastwick Road
Harlow Essex, CM20 2QR(GB)

(54) **Methods of treating bone diseases by administration of a cysteine proteinase inhibitor.**

(57) Methods for treating bone diseases are provided wherein an effective amount of the compound of Formula I:

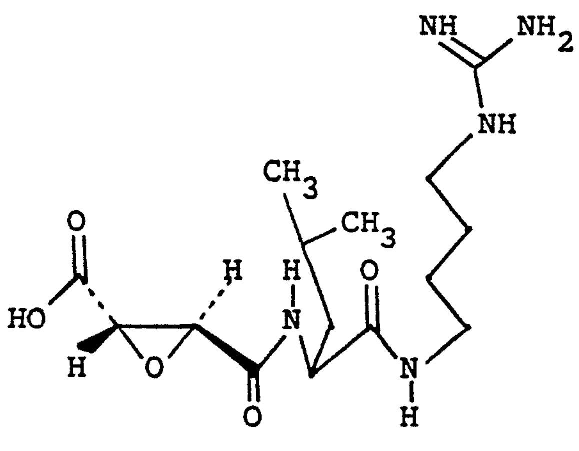

(I)

is administered orally to an animal affected by said bone disease, including malignant hypercalcemia Paget's disease or osteoporosis.

EP 0 269 311 A2

# METHODS OF TREATING BONE DISEASE BY ADMINISTRATION OF A CYSTEINE PROTEINASE INHIBITOR

## BACKGROUND OF THE INVENTION

The pathophysiology of many bone diseases involves the uncoupling of bone resorption and bone formation with a net enhanced bone resportion. Lysosomal enzymes are believed to play a role in bone resorption since the release of a number of acid hydrolases is increased when bone resorption is stimulated by parathyroid hormone. A similar increase in enzyme release occurs when bone resorption is stimulated by prostaglandin E and cyclic adenosine monophosphate. A decrease of release is found when bone resorption is inhibited by calcitonin, colchicine and cortisol. Lysosomal enzymes, therefore, are likely to be a key element in the breakdown of the non-collagenous components of bone matrix, as well as for collagen breakdown.

A direct involvement of lysosomal enzymes in collagen degradation is suggested by the finding that cathepsin B can degrade insoluble collagen. In addition, lysosomal proteases may complete the degradation of collagen which is cleaved into large fragments by the specific collagenase. Moreover, lysosomal proteases may be necessary to cleave intermolecular links between collagen molecules and make them susceptible to degradation by collagenase.

It has recently been shown that several inhibitors of lysosomal cysteine proteases inhibit bone resorption in vitro, [Delaisse et al. , (1984) Biochem. Biophys. Res. Comm., 125, No. 2, p.p. 441-447]. The cysteine proteinases, of which papain is the best known example, contain an essential highly reactive thiol group, and therefore are inhibited by blocking reagents, such as iodoacetate and mercuribenzoate.

A new class of compounds that show promise by acting as class specific inhibitors of cysteine proteinase are L-trans-epoxysuccinyl peptides. One such peptide is the compound L-trans-epoxysuccinyl L-leucylamido (e-guanidino) butane. This compound was shown to inhibit papain with the disappearance of the thiol group of papain and to inhibit cathepsin B, L, and H. By contrast, the serine proteinases and the metalloproteinases were unaffected. It has also been indicated that L-trans-epoxysuccinyl L-leucylamido (e-guanidino) butane inactivates cathepsin B and L by a stoichiometric reaction with the cystein residue essential for catalytic activity [Barrett et al. , Biochem, J. (1982) 201, 189-198]. The kinetics of inhibition also show that the action of L-trans-epoxysuccinyl L-leucylamido (e-guanidino) butane is not competitive with substrate and that the binding is irreversible by a covalent reaction at the active site. The inhibitor is selective due to the affinity of the inhibitors to the appropriate peptide components (dipeptide) for the specificity site of the proteinases.

Certain epoxysuccinyl amino acid derivatives, including L-trans-epoxysuccinyl-L-leucylamido(e-guanidino)butane, (known as E-64), are shown in U.S. Patent 4,418,075 and the art cited therein. The compounds of U.S. Patent 4,418,075, however, are taught to be inhibitors of calcium-activated neutral thiol protease useful in the treatment of muscular dystrophy.

It is, therefore, an object of the present invention to provide a cysteine proteinase inhibitor which is a potent hypocalcemic agents when given orally or intravenously. It is also an object of the present invention to provide an orally active agent for the treatment of diseases associated with increased bone resorption. It is also an object of the present invention to treat diseases associated with increased osteolysis, such as early stages of malignant hypercalcemia, Paget's disease and osteoporosis.

## DESCRIPTION OF THE DRAWINGS

Fig. 1 shows the results on serum $Ca^{++}$ levels over time (14 days) of administration of 10, 25 and 80 mg/kg/day of the formula I compound in a malignant hypercalcemia model.

Fig. 2 shows the results on serum $Ca^{++}$ levels over time (24 hours) of administration of 10, 15 and 40 mg/kg/day of the formula I compound in a model of secondary hyperparathyroidism. Hyperparathyroidism is associated with hypercalcemia resulting from malignant hypercalcemia, Paget's disease and osteoporosis. The 15 and 60 mg/kg/day doses expand the data shown in Table II.

DETAILED DESCRIPTION OF THE INVENTION

In accordance with the present invention, a method of treating bone diseases in animals is provided wherein an effective amount of a compound of the Formula I:

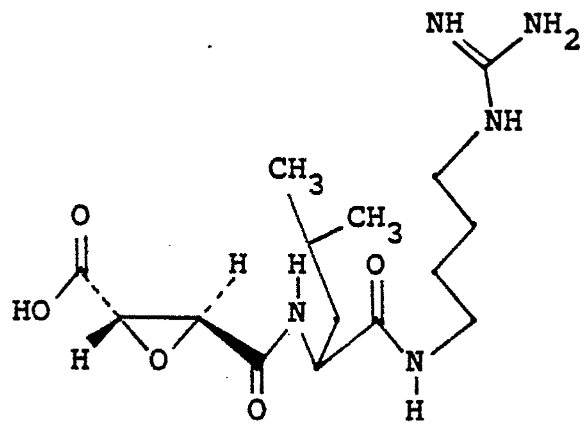

(I)

is administered to an animal affected by said bone disease. The compound of the present invention is particularly effective against bone diseases such as malignant hypercalcimia, Paget's disease, or osteoporosis.

The compound for use in the present invention is epoxysuccinyl-leucylamido (4-guanidino)butane, particularly the L-trans isomer (known as E-64).

Pharmaceutical compositions are provided wherein the active ingredient of the Formula I compound is mixed with a non-toxic pharmaceutical carrier and divided into unit doses, each dose containing a bone disease treating amount of the Formula I compound.

The therapeutic methods of the present invention comprise the administration of effective amounts of the compound of Formula as an active ingredient together with desired pharmaceutically acceptable diluents, adjuvants and carriers to an animal suffering from a bone disease. Unit dosage forms of compound of from 0.1 to 500 mg are administered according to the methods of the invention. Such unit dosage forms may be given to provide a daily dosage of from 1 to 500 mg/kg of body weight of the animal to be treated, preferrably from 10 to 50 mg/kg body weight. Oral administration is the desired route for practice of the invention.

The compound of Formula I is prepared in accordance with the procedures set forth in U.S. Patent 4,418,075, which is hereby incorporated by reference.

The following examples are for illustrative purposes only and are not to be considered as limiting the invention in any way.

EXAMPLE 1

Malignant Hypercalcemia

A rat model that closely mimics the human syndrome of Humoral Hypercalcemia of Malignancy was described by Insogna et al, Endocrinology 114, 888-896 (1984). In this model a Rice 500 Leydig cell tumor is transplated in male Fisher rats. By days 11-14 after transplantation, tumor bearing animals are significantly hypercalcemic. Bone histomorphometry shows uncoupling of bone cell function in the tumor group with marked bone resorption and with marked suppression of bone formation. These findings exactly parallel those in human humoral hypercalcemia of malignancy.

The same model was used to study the effect of the formula I compound on lowering serum $Ca^{++}$ levels in rodents with established malignant hypercalcemia. Rats (150-200 gr) were intubated once a day with three different doses of the formula I compound including 10 mg, 25 mg and 80 mg/kg/day as shown in Figure 1. Control animals received no compound. The compound was given on days 10-15 after tumor transplantation when all animals were hypercalcemic. In experiment No. 2 (Table 1), rodents were intubated once a day with 40 mg/kg, of the formula I compound two days after tumor transplantation. These studies

were utilized to examine whether the formula I compound could prevent tumor-induced hypercalcemia in these animals which generally appears 8-10 days after transplantation.

These studies show that the formula I compound when given orally, can prevent (Table I) or block (Figure 1) tumor induced hypercalcemia.

TABLE I

Effects on Serum Ca++ in Experimental Malignant Hypercalcemia in Rats

Serum mg % $Ca^{++}$

| Days | Untreated | Formula I compound - Treated 40 mg/kg/day |
|---|---|---|
| 2 | 10.1 ± 0.4 | 10.5 ± 0.3 |
| 4 | 9.8 ± 0.3 | 10.1 ± 0.4 |
| 6 | 9.7 ± 0.3 | 10.2 ± 0.2 |
| 8 | 9.6 ± 0.4 | 10.3 ± 0.3 |
| 10 | 10.5 ± 0.3 | 9.8 ± 0.4 |
| 11 | 10.7 ± 0.4 | 10.3 ± 0.4 |
| 12 | 11.2 ± 0.3 | 10.1 ± 0.3 |
| 13 | 12.5 ± 0.3 | 10.4 ± 0.4 |
| 14 | 13.1 ± 0.4 | 10.5 ± 0.3 |
| 15 | 13.3 ± 0.4 | 10.6 ± 0.4 |

EXAMPLE 2

Effect of Formula I Compound on Serum Calcium Levels in a Rat Model for Secondary Hyperparathyroidism

In this model rats are fed a $Ca^{++}$ deficient diet, causing an enhanced release of parathyroid hormone (PTH) and secondary hyperparathyroidism. At this stage most of the serum $Ca^{++}$ is mobilized from bone. To maintain serum calcium levels within the normal range, bone resorption is stimulated by the released parathyroid hormone. This model was used to study the effect of Formula I compound on rats with secondary hyperparathyroidism. Rats (150-200 gr) were kept on a $Ca^{++}$ deficient diet for 7 days. Different doses of Formula I compound were intubated into animals (n = 10) and serum $Ca^{++}$ was monitored at 0, 4, 8, 12 and 24 hours. The most effective dose 40 mg/kg caused a drop of a significant 28.8% in 12 hours (Table No. II and Figure 2).

## TABLE II

### Effect on Serum $Ca^{++}$ in Experimental Secondary Hyperparathyroidism in Rats

Serum mg % $Ca^{++}$

Formula I Compound Treatment

| Hours | 10 mg | 20 mg | 60 mg |
|---|---|---|---|
| 0 | 11.5 ± 0.5 | 11.3 ± 0.4 | 11.7 ± 0.6 |
| 4 | 10.6 ± 0.4 | 10.2 ± 0.3 | 9.8 ± 0.4 |
| 8 | 10.4 ± 0.5 | 9.7 ± 0.4 | 9.5 ± 0.3 |
| 12 | 10.2 ± 0.4 | 9.5 ± 0.3 | 8.9 ± 0.4 |
| 24 | 11.5 ± 0.6 | 12.0 ± 0.3 | 12.7 ± 0.4 |

## Claims

1. The use of a compound of the formula:

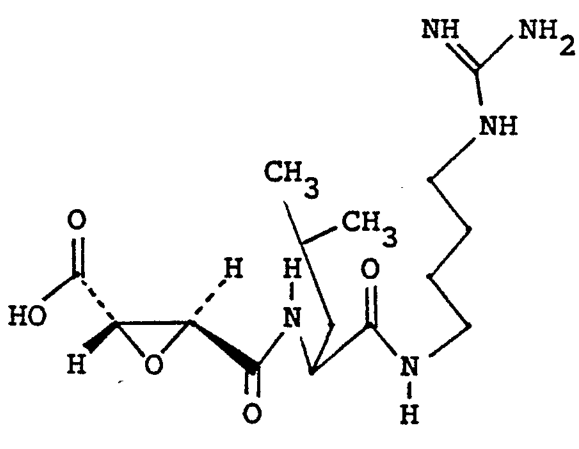

(I)

for the preparation of a medicament useful for treating bone diseases.

2. The use as claimed in Claim 1, wherein said bone diseases are malignant hypercalcemia, Paget's disease or osteoporosis.

3. The use as claimed in Claim 2, wherein the compound is administered at a dosage of from 1 to 500 mg/kg of body weight.

4. The use as claimed in Claim 3, wherein the compound is administered at a dosage of from 10 to 50 mg/kg of body weight.

5. The use as claimed in Claim 4, wherein the compound is administered orally.

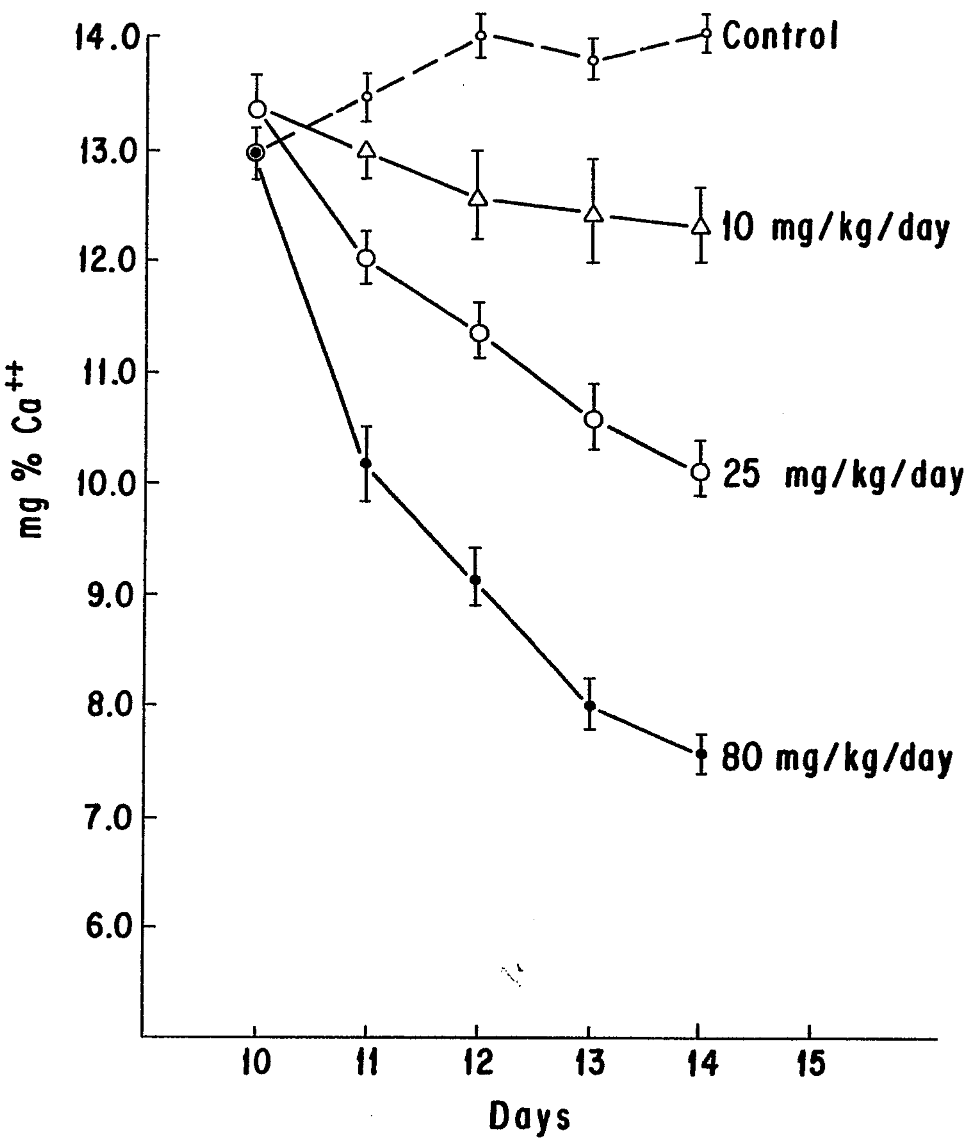


MALIGNANT HYPERCALCEMIA
ORAL ADMINISTRATION OF E-64
DOSE (MG/KG/DAY)

FIG. 1

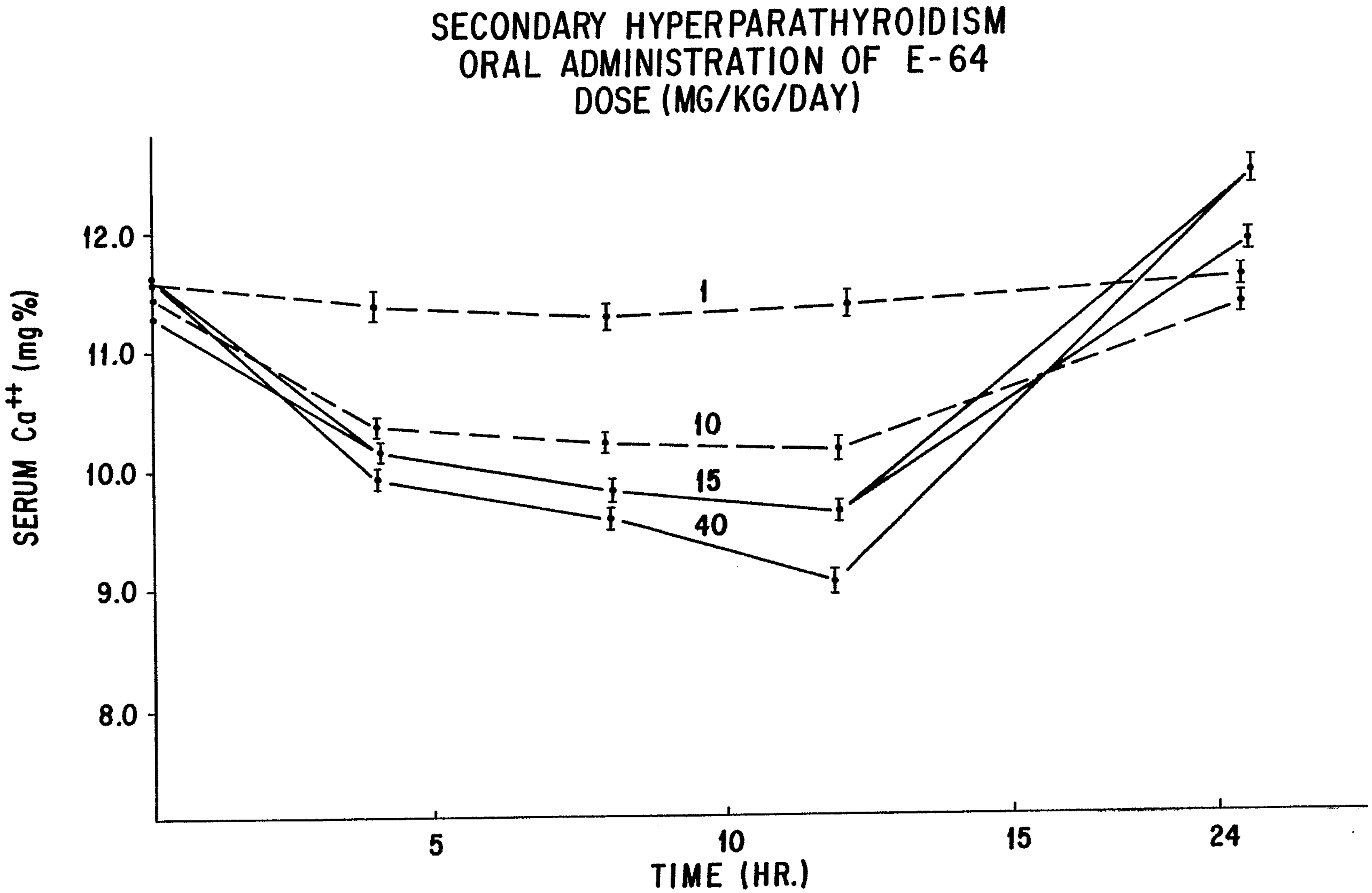
SECONDARY HYPERPARATHYROIDISM
ORAL ADMINISTRATION OF E-64
DOSE (MG/KG/DAY)
SERUM $Ca^{++}$ (mg %)
12.0
11.0
10.0
9.0
8.0
1
10
15
40
5
10
15
24
TIME (HR.)

FIG. 2